(19) Europäisches Patentamt
European Patent Office
Office européen des brevets

(11) **EP 4 488 662 A1**

(12) **EUROPEAN PATENT APPLICATION**
published in accordance with Art. 153(4) EPC

(43) Date of publication:
**08.01.2025   Bulletin 2025/02**

(21) Application number: **23784774.4**

(22) Date of filing: **05.04.2023**

(51) International Patent Classification (IPC):
*G01N 21/45* (2006.01)    *C12M 1/34* (2006.01)
*C12Q 1/02* (2006.01)    *G02B 21/36* (2006.01)

(52) Cooperative Patent Classification (CPC):
**C12M 1/34; C12Q 1/02; G01N 21/45; G02B 21/36**

(86) International application number:
**PCT/JP2023/014105**

(87) International publication number:
**WO 2023/195490 (12.10.2023 Gazette 2023/41)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **06.04.2022   JP 2022063639**

(71) Applicant: **FUJIFILM Corporation
Tokyo 106-8620 (JP)**

(72) Inventor: **YAMAMOTO, Hiroaki
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **IMAGING SYSTEM AND CELL CONCENTRATION ADJUSTMENT METHOD**

(57)    An imaging system includes: a light source that emits light toward a flow channel through which a sample containing a cell flows; an imaging sensor that generates an interference fringe image including an interference fringe by imaging light that has passed through the flow channel; and a processor that estimates a cell concentration in the sample based on the interference fringe image or a reconstructed image obtained by reconstructing the interference fringe image, and adjusts the cell concentration in the sample based on an estimated value of the cell concentration.

FIG. 9

EP 4 488 662 A1

**Description**

BACKGROUND OF THE INVENTION

1. Field of the Invention

The technology of the present disclosure relates to an imaging system and a cell concentration adjustment method.

2. Description of the Related Art

In order to reduce a size of a device that images a small object to be observed, such as a cell, so-called lens-free digital holography in which an optical-system component is eliminated is known. In the digital holography, the object to be observed is imaged by using a light source that emits coherent light such as a laser beam, and an interference fringe image including an interference fringe image obtained by the imaging is reconstructed to generate a reconstructed image at a predetermined reconstruction position (see, for example, WO2018/158947A).

In addition, inline sensing is known, which makes it possible to monitor a culture state of cells in real time by imaging a microchannel while a sample containing cells flows from a culture tank to the microchannel.

SUMMARY OF THE INVENTION

The sample flowing through the microchannel may experience a change in cell concentration. In a case where the cell concentration is high, an amount of light scattered by the cells increases, and the interference fringe image is affected by scattering. In a case where the interference fringe image is affected by scattering, correct information about the cell cannot be obtained, and as a result, the reconstructed image deteriorates.

In a cell observation device described in WO2018/158947A, in a case where an operator determines that measurement is not appropriate by looking at the interference fringe image, the operator can immediately stop the measurement by pressing a measurement stop button. As a result, in a case where there is a problem in the measurement, such as the presence of a foreign substance mixed in the sample, it is possible to avoid consuming unnecessary reconstruction time or the like.

However, in the inline sensing, it is necessary to monitor the culture state of the cells while continuously observing a large amount of samples flowing through the flow channel. Therefore, as described in WO2018/158947A, a method of determining appropriateness of the measurement by the operator is not practical because the burden on the operator is significant.

An object of the technology of the present disclosure is to provide an imaging system and a cell concentration adjustment method that make it possible to suppress deterioration of a reconstructed image due to a change in cell concentration.

In order to achieve the above object, according to an aspect of the present disclosure, there is provided an imaging system comprising: a light source that emits light toward a flow channel through which a sample containing a cell flows; an imaging sensor that generates an interference fringe image including an interference fringe by imaging light that has passed through the flow channel; and a processor that estimates a cell concentration in the sample based on the interference fringe image or a reconstructed image obtained by reconstructing the interference fringe image, and adjusts the cell concentration in the sample based on an estimated value of the cell concentration.

It is preferable that the processor estimates the cell concentration based on the number of the interference fringes included in the interference fringe image or frequency information of the interference fringe image.

It is preferable that the processor estimates the cell concentration based on an image quality of the reconstructed image.

It is preferable that the processor estimates the cell concentration based on a brightness or a brightness distribution of the entire interference fringe image.

It is preferable that the processor adjusts the cell concentration in the sample by controlling a pump that introduces the sample from a culture tank into the flow channel.

It is preferable that the processor adjusts the cell concentration in the sample by controlling at least one of a diameter of a suction port of the pump, a position of the suction port, or a suction rate.

It is preferable that the processor adjusts the cell concentration in the sample by controlling an injection amount of a diluent to be injected into the flow channel.

It is preferable that, in a case where the estimated value is higher than an appropriate range, the processor changes the cell concentration stepwise until the estimated value is within the appropriate range.

It is preferable that, in a case where the estimated value is lower than the appropriate range, the processor changes the cell concentration stepwise until the estimated value is within the appropriate range.

It is preferable that the processor performs control to notify of an abnormality in a case where the estimated value is not within the appropriate range even after the cell concentration is changed a certain number of times.

According to another aspect of the present disclosure, there is provided a cell concentration adjustment method comprising: emitting light toward a flow channel through which a sample containing a cell flows; generating an interference fringe image including an interference fringe by imaging light that has passed through the flow channel; and estimating a cell concentration in the sample based on the interference fringe image or a reconstructed image obtained by reconstructing the interference fringe image, and adjusting the cell concentration in the sample based on an estimated value of the cell concentration.

According to the technology of the present disclosure, it is possible to provide an imaging system and a cell concentration adjustment method that make it possible to suppress deterioration of a reconstructed image due to a change in cell concentration.

BRIEF DESCRIPTION OF THE DRAWINGS

Fig. 1 is a diagram showing an example of a configuration of a cell culture system according to an embodiment.
Fig. 2 is a diagram showing an example of a configuration of a cell culture device.
Fig. 3 is a diagram showing a state in which an interference fringe is generated by a cell.
Fig. 4 is a diagram showing an example of an interference fringe image.
Fig. 5 is a block diagram showing an example of a hardware configuration of an information processing apparatus.
Fig. 6 is a block diagram showing an example of a functional configuration of the information processing apparatus.
Fig. 7 is a diagram conceptually showing an example of the interference fringe image in a case where a cell concentration is high.
Fig. 8 is a diagram conceptually showing an example of control of the cell concentration by the cell culture device.
Fig. 9 is a flowchart showing an example of a flow of an operation of an imaging system.
Fig. 10 is a graph conceptually showing an example of a change in brightness distribution due to a change in cell concentration.
Fig. 11 is a block diagram showing a functional configuration of an information processing apparatus according to a fourth modification example.
Fig. 12 is a flowchart showing an example of a flow of an operation of an imaging system according to the fourth modification example.
Fig. 13 is a diagram showing a configuration of a cell culture system according to a fifth modification example.
Fig. 14 is a flowchart showing a flow of an operation of an imaging system according to a sixth modification example.

DESCRIPTION OF THE PREFERRED EMBODIMENTS

An example of an embodiment according to the technology of the present disclosure will be described with reference to the accompanying drawings.

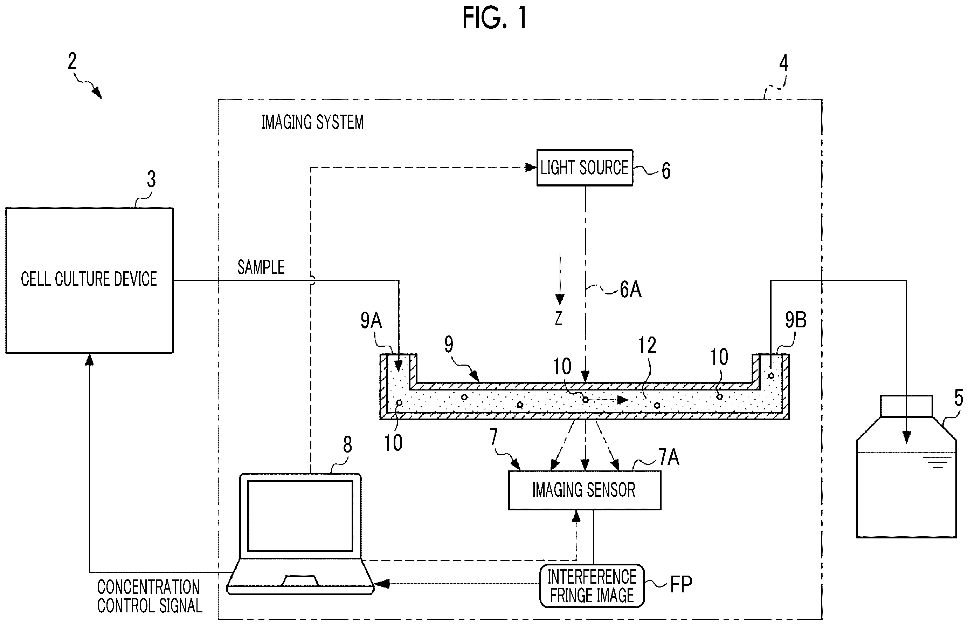

Fig. 1 shows an example of a configuration of a cell culture system 2 according to the embodiment. The cell culture system 2 includes a cell culture device 3, an imaging system 4, and a recovery tank 5. The imaging system 4 includes a light source 6, an imaging sensor 7, and an information processing apparatus 8. The information processing apparatus 8 is, for example, a personal computer.

The light source 6 is, for example, a laser diode. In addition, the light source 6 may be configured by combining a light emitting diode and a pinhole.

A microchannel 9 is disposed between the light source 6 and the imaging sensor 7. The microchannel 9 is formed in, for example, a channel unit formed of a silicone resin and is a flow channel through which a liquid can flow. The channel unit is transparent to light and can irradiate the inside of the microchannel 9 with light from the outside of the channel unit. The microchannel 9 is provided with an opening portion 9A for introducing a sample 12 which is a solution containing a cell 10, and an opening portion 9B for discharging the sample 12 introduced into the microchannel 9. The microchannel 9 is an example of a "flow channel" according to the technology of the present disclosure.

The sample 12 is introduced from the cell culture device 3 into the opening portion 9A of the microchannel 9, flows through the microchannel 9 at a constant rate, and is discharged from the opening portion 9B. The sample 12 discharged from the opening portion 9B is recovered by the recovery tank 5.

The light source 6, the imaging sensor 7, and the microchannel 9 are disposed in, for example, an incubator (not shown). For example, the imaging system 4 targets one or more cells 10 included in the sample 12 to be imaged, and images the light emitted from the light source 6 and passed through the microchannel 9 by using the imaging sensor 7.

The light source 6 emits irradiation light 6A toward the microchannel 9. The irradiation light 6A is coherent light, and is, for example, a laser beam. The irradiation light 6A is incident on the microchannel 9, transmits through the microchannel 9, and then is incident on an imaging surface 7A of the imaging sensor 7. A Z direction indicated by an arrow is an irradiation direction of the irradiation light 6A. The microchannel 9 is disposed such that a flow direction of the sample 12 is substantially perpendicular to the Z direction.

A shape of the microchannel 9 and the number of opening portions 9A and 9B can be changed as appropriate. Further, the number of microchannels 9 disposed between the light source 6 and the imaging sensor 7 is not limited to one and may be two or more. In the present embodiment, one microchannel 9 is assumed to be disposed between the light source 6 and the imaging sensor 7.

The imaging sensor 7 is composed of, for example, a monochrome complementary metal oxide semiconductor (CMOS) image sensor. An imaging operation of the imaging sensor 7 is controlled by the information processing apparatus 8. The imaging sensor 7 is disposed such that the imaging surface 7A is perpendicular to the Z direction. The imaging surface 7A may be in contact with the channel unit in which the microchannel 9 is formed.

The irradiation light 6A is incident on the sample 12 in the microchannel 9 and diffracted by the cell 10, and thus an interference fringe reflecting a shape of the cell 10 is generated. The imaging sensor 7 generates an interference fringe image FP which includes the interference fringe and outputs the interference fringe image FP to the information processing apparatus 8.

The information processing apparatus 8 performs imaging control, generation of a reconstructed image based on the interference fringe image FP, estimation of a concentration (hereinafter referred to as a cell concentration) of the cells 10 in the sample 12 based on the interference fringe image FP, and control of the cell culture device 3 based on an estimated value of the cell concentration.

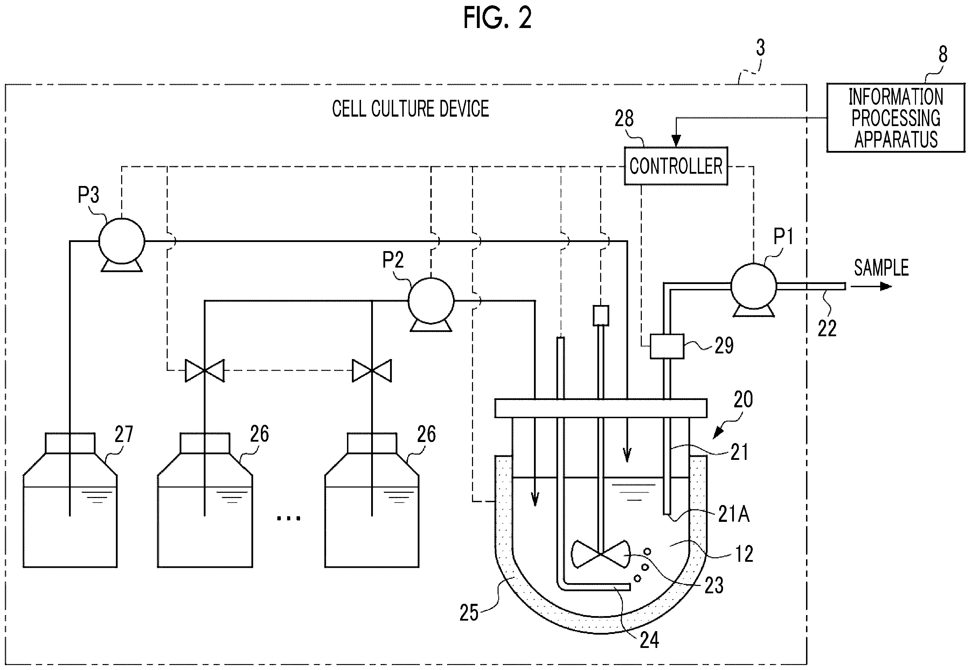

Fig. 2 shows an example of a configuration of the cell culture device 3. The cell culture device 3 comprises a culture tank 20, a suction pump P1, a suction pipe 21, a transfer pipe 22, a stirring device 23, a ventilation device 24, a temperature adjusting device 25, a culture medium tank 26, a culture medium pump P2, a solution tank 27, a solution pump P3, and a controller 28. The controller 28 controls the suction pump P1, the stirring device 23, the ventilation device 24, the temperature adjusting device 25, and the solution pump P3. The controller 28 is controlled by the information processing apparatus 8. The suction pump P1 is an example of a "pump" according to the technology of the present disclosure.

The culture tank 20 is a tank for culturing the cell 10 and accommodates the sample 12 containing the cell 10. One end of the suction pipe 21 is inserted into the culture tank 20, and the other end thereof is connected to the suction pump P1. The suction pump P1 suctions the sample 12 from a suction port 21A provided at the one end of the suction pipe 21. One end of the transfer pipe 22 is connected to the suction pump P1, and the other end thereof is connected to the opening portion 9A of the microchannel 9. The transfer pipe 22 is a pipe for transferring the sample 12 suctioned by the suction pump P1 and introducing the sample 12 into the microchannel 9.

An actuator 29 that operates based on control from the controller 28 is connected to the suction pipe 21. The actuator 29 moves the suction pipe 21 to change a position of the suction port 21A in the culture tank 20. In addition, the suction pipe 21 is configured such that a diameter of the suction port 21A can be changed. The actuator 29 changes the diameter of the suction port 21A based on the control from the controller 28. Here, the diameter of the suction port 21A means an inner diameter of the suction port 21A.

The stirring device 23 stirs the sample 12 in the culture tank 20 by rotating a stirring blade. The ventilation device 24 ventilates the sample 12 in the culture tank 20 with air, oxygen, nitrogen, carbon dioxide, or the like. The temperature adjusting device 25 adjusts a temperature of the sample 12 in the culture tank 20.

The culture medium tank 26 is a tank for accommodating a liquid culture medium. The culture medium pump P2 delivers the culture medium in the culture medium tank 26 to the culture tank 20. A liquid delivery amount by the culture medium pump P2 is controlled by the controller 28 according to a concentration of culture medium components in the culture medium tank 26 and the like. In the present embodiment, a plurality of the culture medium tanks 26 are connected to the culture medium pump P2.

The solution tank 27 is a tank for accommodating an alkaline solution. The solution pump P3 delivers the alkaline solution in the solution tank 27 to the culture tank 20. The pH of the sample 12 is controlled within a predetermined range by addition of an alkaline solution, ventilation with carbon dioxide, or the like.

In addition, various sensors (not shown) are provided in the culture tank 20 to monitor a culture environment in the culture tank 20 and a culture state of the cell 10. For example, the culture tank 20 is provided with a temperature sensor such as a thermocouple, a dissolved oxygen sensor, a carbon dioxide sensor, a pH sensor, a cell counter, and the like.

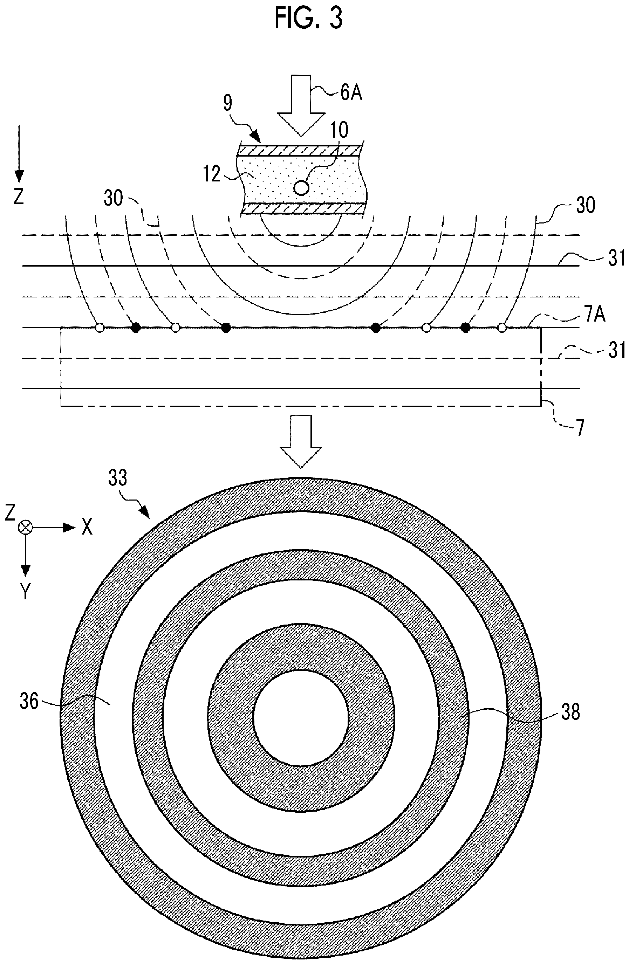

Fig. 3 shows a state in which the interference fringe is generated by the cell 10 included in the sample 12 that flows through the microchannel 9. A part of the irradiation light 6A incident on the microchannel 9 is diffracted by the cell 10. That is, the irradiation light 6A is divided into diffracted light 30 diffracted by the cell 10 and transmitted light 31 that is not diffracted by the cell 10 and transmits through the microchannel 9. The transmitted light 31 is a planar wave. The diffracted light 30 and the transmitted light 31 transmit through the microchannel 9 and are incident on the imaging surface 7A of the imaging sensor 7.

The diffracted light 30 and the transmitted light 31 interfere with each other to generate an interference fringe 33. The interference fringe 33 is composed of a bright portion 36 and a dark portion 38. In Fig. 3, the bright portion 36 and the dark portion 38 of the interference fringe 33 are shown as circular portions, respectively. However, a shape of the interference fringe 33 changes according to a shape and an internal structure of the cell 10. The imaging sensor 7 captures a light image including the interference fringe 33 formed on the imaging surface 7A and outputs the interference fringe image FP including the interference fringe 33.

In addition to the diffracted light 30 and the transmitted light 31, light components generated by the incidence of the irradiation light 6A on the sample 12 include scattered light generated by the irradiation light 6A being scattered by the cell 10 or the like. The interference fringe image FP deteriorates depending on an amount of the scattered light. In a case where the interference fringe image FP deteriorates, the reconstructed image generated based on the interference fringe image FP also deteriorates.

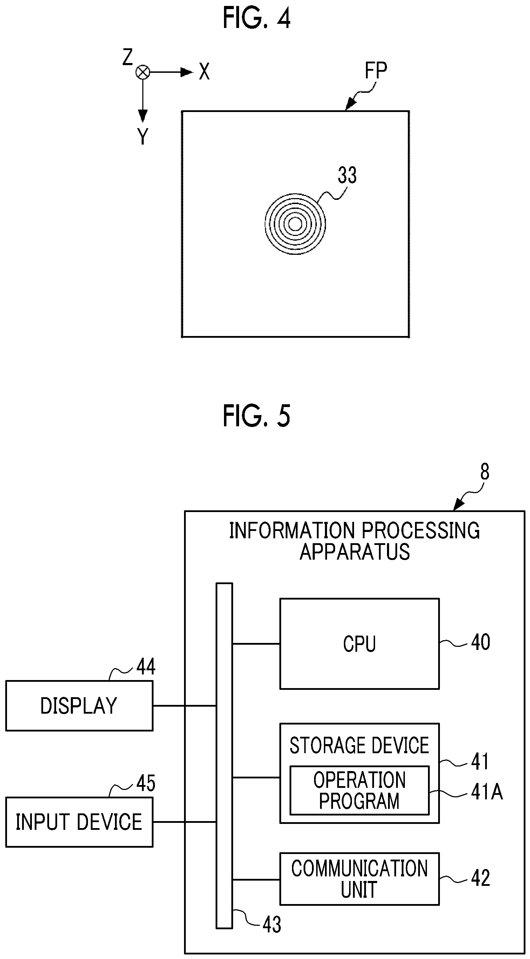

Fig. 4 shows an example of the interference fringe image FP output from the imaging sensor 7. The interference fringe image FP shown in Fig. 4 includes one interference fringe 33 generated by the diffraction of the irradiation light 6A by one cell 10 included in an imaging region of the imaging sensor 7.

Fig. 5 shows an example of a hardware configuration of the information processing apparatus 8. As shown in Fig. 5, the information processing apparatus 8 comprises a central processing unit (CPU) 40, a storage device 41, and a communication unit 42, which are interconnected via a bus line 43. In addition, a display 44 and an input device 45 are connected to the bus line 43. The input device 45 is a keyboard, a mouse, a touch panel, or the like, and is operated by a user in a case of inputting information.

The CPU 40 is a computation device that reads out an operation program 41A and various types of data (not shown) stored in the storage device 41 and executes processing to realize various functions. The CPU 40 is an example of a "processor" according to the technology of the present disclosure.

The storage device 41 includes, for example, a random access memory (RAM), a read only memory (ROM), or a storage device. The RAM is, for example, a volatile memory used as a work area or the like. The ROM is, for example, a non-volatile memory such as a flash memory that holds the operation program 41A and various types of data. The storage device is, for example, a hard disk drive (HDD) or a solid state drive (SSD). The storage device stores an operating system (OS), an application program, image data, various types of data, and the like.

The communication unit 42 is a network interface that controls transmission of various types of information via a network such as a local area network (LAN) or a wide area network (WAN). The information processing apparatus 8 is connected to the light source 6, the imaging sensor 7, and the cell culture device 3 via the communication unit 42. The display 44 displays various screens. The information processing apparatus 8 receives input of an operation instruction from the input device 45 through various screens.

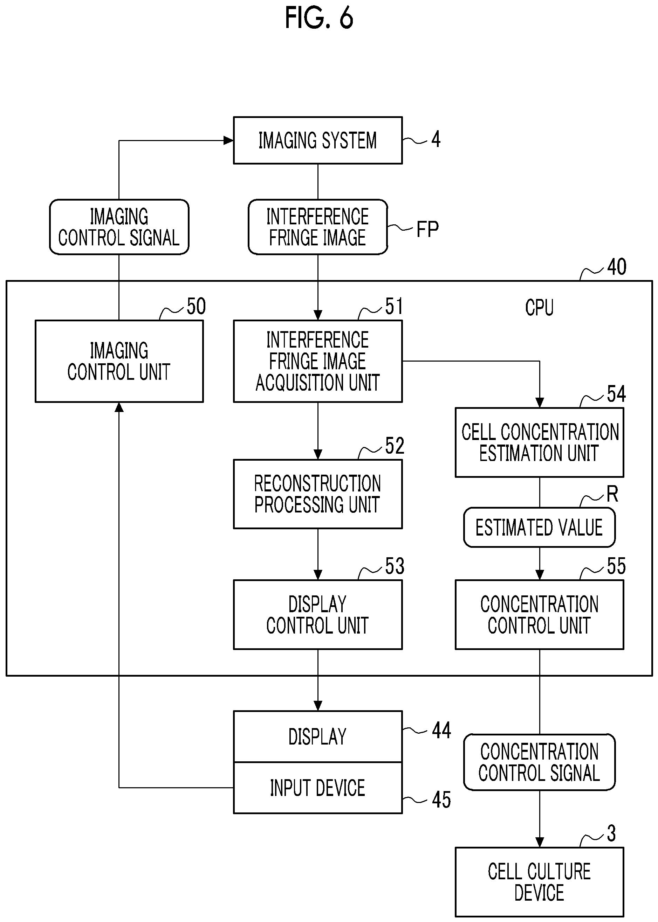

Fig. 6 shows an example of a functional configuration of the information processing apparatus 8. A function of the information processing apparatus 8 is realized by the CPU 40 executing processing based on the operation program 41A. As shown in Fig. 6, the CPU 40 comprises an imaging control unit 50, an interference fringe image acquisition unit 51, a reconstruction processing unit 52, a display control unit 53, a cell concentration estimation unit 54, and a concentration control unit 55.

The imaging control unit 50 controls an operation of the imaging system 4. Specifically, the imaging control unit 50 controls a generation operation of the irradiation light 6A by the light source 6 and an imaging operation of the imaging sensor 7 by transmitting an imaging control signal to the imaging system 4. Hereinafter, the generation operation of the irradiation light 6A by the light source 6 and the imaging operation of the imaging sensor 7 are collectively referred to as an imaging operation of the imaging system 4. The imaging control unit 50 causes the imaging system 4 to initiate the imaging operation based on an operation signal input from the input device 45.

The interference fringe image acquisition unit 51 acquires the interference fringe image FP output from the imaging sensor 7. The interference fringe image acquisition unit 51 supplies the acquired interference fringe image FP to the reconstruction processing unit 52.

The reconstruction processing unit 52 generates a reconstructed image by performing computation based on the interference fringe image FP. For example, the reconstruction processing unit 52 generates the reconstructed image each time a reconstruction position is changed while changing the reconstruction position by a certain value in the Z direction. The reconstruction position P is a position (so-called depth position) represented by a distance d from the imaging surface 7A of the imaging sensor 7 in a direction of the light source 6. Hereinafter, the reconstruction position is also referred to as a focal position.

The reconstruction processing unit 52 performs reconstruction processing based on, for example, Fresnel transform equations represented by the following equations (1) to (3).

$$\Gamma(m,n) = \frac{i}{\lambda d}\exp\left(-i\frac{2\pi}{\lambda}d\right)\exp\left[-i\pi\lambda d\left(\frac{m^2}{N_x^2\Delta x^2} + \frac{n^2}{N_y^2\Delta y^2}\right)\right]$$

$$\times \sum_{x=0}^{N_x-1}\sum_{y=0}^{N_y-1} I(x,y)\exp\left[-i\frac{\pi}{\lambda d}(x^2\Delta x^2 + y^2\Delta y^2)\right]\exp\left[i2\pi\left(\frac{xm}{N_x^2} + \frac{yn}{N_y^2}\right)\right] \quad\cdots(1)$$

$$A_0(m,n) = |\Gamma(m,n)|^2 \quad\cdots(2)$$

$$\varphi_0(m,n) = \arctan\frac{\mathrm{Im}[\Gamma(m,n)]}{\mathrm{Re}[\Gamma(m,n)]} \quad\cdots(3)$$

Here, I(x,y) represents image data. x represents coordinates of pixels constituting the imaging sensor 7 in the X direction. y represents coordinates of the pixels in the Y direction. $\Delta x$ is an arrangement pitch of the pixels in the X direction. $\Delta y$ is an arrangement pitch of the pixels in the Y direction. $\lambda$ is a wavelength of the irradiation light 6A.

As shown in Equation (1), $\Gamma(m,n)$ is a complex amplitude image obtained by performing the Fresnel transform on the interference fringe included in the interference fringe image FP. Here, m = 1, 2, 3, ... Nx-1, and n = 1, 2, 3, ... Ny-1. Nx represents the number of pixels of the interference fringe image FP in the X direction. Ny represents the number of pixels of the interference fringe image FP in the Y direction.

As shown in Equation (2), $A_0(m, n)$ is an amplitude image representing amplitude components (that is, intensity components) of the complex amplitude image $\Gamma(m, n)$. As shown in Equation (3), $\varphi_0(m,n)$ represents a phase difference image representing phase difference components of the complex amplitude image $\Gamma(m,n)$.

The reconstruction processing unit 52 obtains the complex amplitude image $\Gamma(m,n)$ based on Equation (1), and applies the obtained complex amplitude image $\Gamma(m,n)$ to Equation (2) or Equation (3) to obtain the amplitude image $A_0(m,n)$ or the phase difference image $\varphi_0(m,n)$. The reconstruction processing unit 52 outputs at least one of the amplitude image $A_0(m,n)$ or the phase difference image $\varphi_0(m,n)$ as the reconstructed image.

The reconstruction processing unit 52 is not limited to a method using the Fresnel transform equation, and may perform the reconstruction processing by a Fourier iterative phase retrieval method or the like.

The display control unit 53 causes the display 44 to display the reconstructed image generated by the reconstruction processing unit 52.

The cell concentration estimation unit 54 estimates the cell concentration in the sample 12 flowing through the microchannel 9 based on the interference fringe image FP acquired by the interference fringe image acquisition unit 51. In the present embodiment, the cell concentration estimation unit 54 estimates the cell concentration based on the number of the interference fringes 33 included in the interference fringe image FP. The cell concentration estimation unit 54 estimates that the cell concentration is higher as the number of the interference fringes 33 increases. The cell concentration estimation unit 54 outputs an estimated value R of the cell concentration to the concentration control unit 55.

In the present disclosure, the estimated value R may not be a numerical value directly representing the cell concentration, and may be a numerical value related to the cell concentration. In the present embodiment, the number of the interference fringes 33 included in the interference fringe image FP is used as the estimated value R.

The concentration control unit 55 determines whether or not the estimated value R output from the cell concentration estimation unit 54 is within an appropriate range. In a case where it is determined that the estimated value R is not within the appropriate range, the concentration control unit 55 adjusts the cell concentration by outputting a concentration control signal to the cell culture device 3.

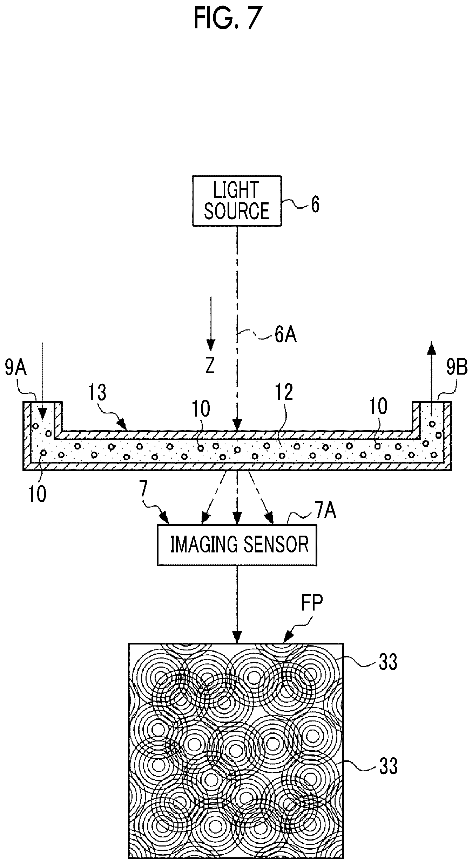

Fig. 7 conceptually shows an example of the interference fringe image FP in a case where the cell concentration is high. In a case where the cell concentration is high, a plurality of the interference fringes 33 appear in the interference fringe image FP. In a case where the cell concentration is high, an amount of light scattered by each cell 10 increases, and the interference fringe image FP deteriorates. As a result, the reconstructed image deteriorates.

The concentration control unit 55 controls the cell culture device 3 to decrease the cell concentration to be within the appropriate range in a case where the estimated value R is higher than the appropriate range. The appropriate range refers to a range of the cell concentration in which the deterioration of the reconstructed image is permissible. The concentration control unit 55 controls the cell culture device 3 to increase the cell concentration to be within the appropriate range in a case where the estimated value R is lower than the appropriate range.

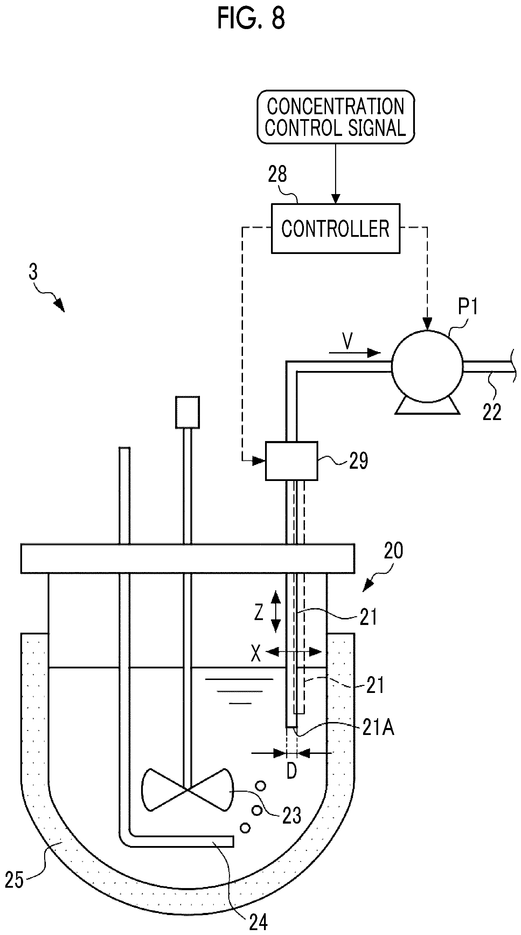

Fig. 8 conceptually shows an example of the control of the cell concentration by the cell culture device 3. The cell concentration in the sample 12 in the culture tank 20 is considered to change depending on the diameter of the suction port 21A, the position of the suction port 21A, and a suction rate V of the sample 12 by the suction pump P1. Since the sample 12 in the culture tank 20 is stirred by the stirring device 23, the cell concentration varies depending on the position in the culture tank 20.

Based on the concentration control signal input from the concentration control unit 55, the controller 28 controls the actuator 29 to change the diameter D of the suction port 21A or the position of the suction port 21A, and controls the suction pump P1 to change the suction rate V. The position of the suction port 21A is related to the X direction, the Y direction, and the Z direction. For example, the controller 28 changes the cell concentration by a predetermined amount designated by the concentration control signal by changing at least one of the diameter D of the suction port 21A, the position of the suction port 21A, or the suction rate V with reference to a detection value of the cell concentration by the cell counter. The controller 28 may change the position of the suction port 21A or the like based on distribution data of the cell concentration in the culture tank 20 acquired in the past through experiments or the like.

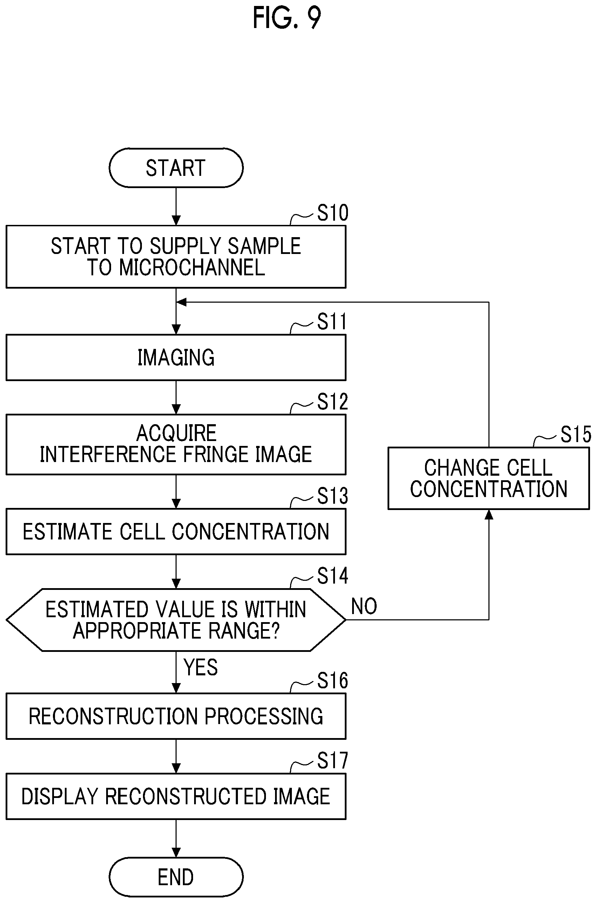

Fig. 9 shows an example of a flow of an operation of the imaging system 4. First, the cell culture device 3 starts to supply the sample 12 to the microchannel 9 (step S10). Next, the imaging control unit 50 causes the imaging system 4 to perform an imaging operation (step S11). The interference fringe image acquisition unit 51 acquires the interference fringe image FP output from the imaging sensor 7 (step S12).

The cell concentration estimation unit 54 estimates the cell concentration based on the interference fringe image FP acquired by the interference fringe image acquisition unit 51, and outputs the estimated value R to the concentration control unit 55 (step S13). The concentration control unit 55 determines whether or not the estimated value R is within the appropriate range (step S14). In a case where the estimated value R is not within the appropriate range (step S14: NO), the concentration control unit 55 changes the cell concentration by outputting the concentration control signal to the cell culture device 3 (step S15). After step S15, the processing returns to step S11.

The processing of steps S11 to S15 is repeatedly performed until the estimated value R is determined to be within the appropriate range in step S14. That is, the adjustment of the cell concentration is performed by changing the cell concentration stepwise. Specifically, in a case where the estimated value R is higher than the appropriate range, the cell concentration is changed stepwise until the cell concentration is within the appropriate range. Even in cases where the estimated value R is lower than the appropriate range, the cell concentration is changed stepwise until the cell concentration is within the appropriate range.

In a case where it is determined that the estimated value R is within the appropriate range (step S14: YES), the reconstruction processing unit 52 generates the reconstructed image by performing the reconstruction processing based on the interference fringe image FP (step S16). The display control unit 53 causes the display 44 to display the reconstructed image generated by the reconstruction processing unit 52 (step S17).

After step S17, the processing may return to step S11, and steps S11 to S17 may be repeatedly executed.

As described above, according to the technology of the present disclosure, the cell concentration in the sample is estimated based on the interference fringe image, and the cell concentration in the sample is adjusted based on the estimated value. Therefore, it is possible to suppress the deterioration of the reconstructed image due to the change in the cell concentration.

Next, various modification examples of the above-described embodiment will be described. In the following modification examples, only points different from the above-described embodiment will be described.

[First Modification Example]

In the above-described embodiment, the cell concentration estimation unit 54 estimates the cell concentration based on the number of the interference fringes 33 included in the interference fringe image FP. Alternatively, the cell concentration estimation unit 54 may estimate the cell concentration based on frequency information of the interference fringe image FP by performing frequency analysis on the interference fringe image FP.

As the cell concentration increases, high-frequency components due to light scattering increase in the interference fringe image FP. Further, as the cell concentration increases, the interference fringe 33 disappears due to a decrease in coherence, and the high-frequency components due to light scattering become dominant. The cell concentration estimation unit 54 can estimate the cell concentration by analyzing the frequency components included in the interference fringe image FP.

[Second Modification Example]

The cell concentration estimation unit 54 may estimate the cell concentration based on a brightness of the entire interference fringe image FP. For example, the cell concentration estimation unit 54 obtains an integrated brightness or an average brightness of a plurality of pixels constituting the interference fringe image FP, and estimates the cell concentration based on the integrated brightness or the average brightness. As the cell concentration increases, a light intensity of the irradiation light 6A passing through the microchannel 9 decreases, and an amount of light incident on the imaging sensor 7 decreases. As described above, since there is a correlation between the cell concentration and the brightness of the entire interference fringe image FP, the cell concentration estimation unit 54 can estimate the cell concentration based on the brightness of the entire interference fringe image FP.

[Third Modification Example]

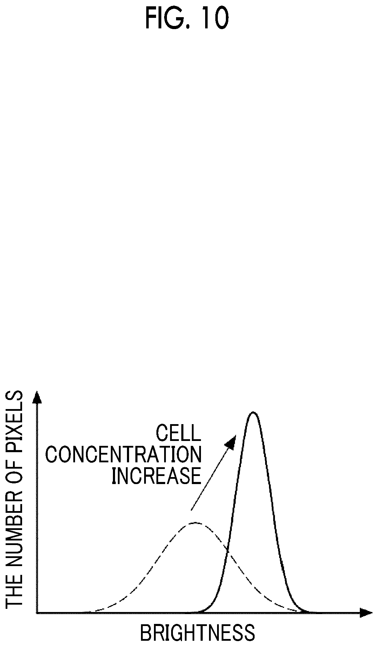

The cell concentration estimation unit 54 may estimate the cell concentration based on a brightness distribution of the entire interference fringe image FP. For example, the cell concentration estimation unit 54 estimates the cell concentration based on a variance value of the brightness distribution representing a relationship between the brightness and the number of pixels. As shown in Fig. 10, it is considered that, as the cell concentration increases, the high-frequency components due to light scattering increase in the interference fringe image FP, and the interference fringe 33 becomes blurred, resulting in a decrease in the variance value of the brightness distribution. As described above, it is considered that there is a correlation between the cell concentration and the brightness distribution. Therefore, the cell concentration estimation unit 54 can estimate the cell concentration based on the brightness distribution of the entire interference fringe image FP.

[Fourth Modification Example]

In the above-described embodiment, the cell concentration estimation unit 54 estimates the cell concentration based on the interference fringe image FP. Alternatively, the cell concentration estimation unit 54 may estimate the cell concentration based on the reconstructed image obtained by reconstructing the interference fringe image FP.

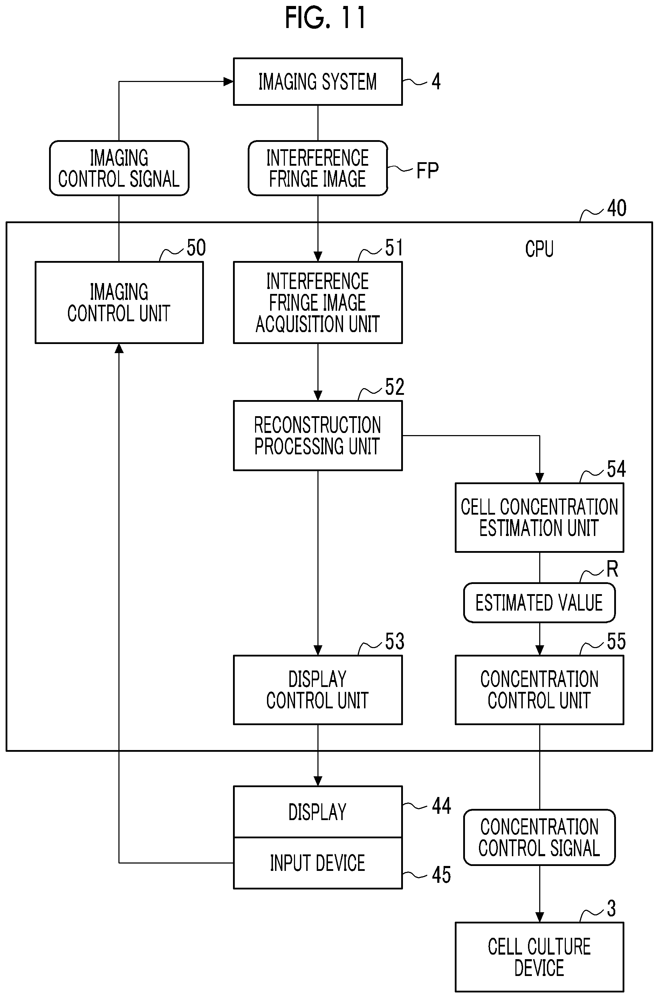

Fig. 11 shows a functional configuration of the information processing apparatus 8 according to a fourth modification example. In the present modification example, the cell concentration estimation unit 54 estimates the cell concentration based on the interference fringe image FP generated by the reconstruction processing unit 52. As the cell concentration increases, the amount of light scattered increases so that an image quality of the reconstructed image deteriorates. Therefore, in the present modification example, the cell concentration estimation unit 54 estimates the cell concentration based on the image quality of the reconstructed image. The image quality refers to, for example, a sharpness of a contour of the cell appearing in the reconstructed image.

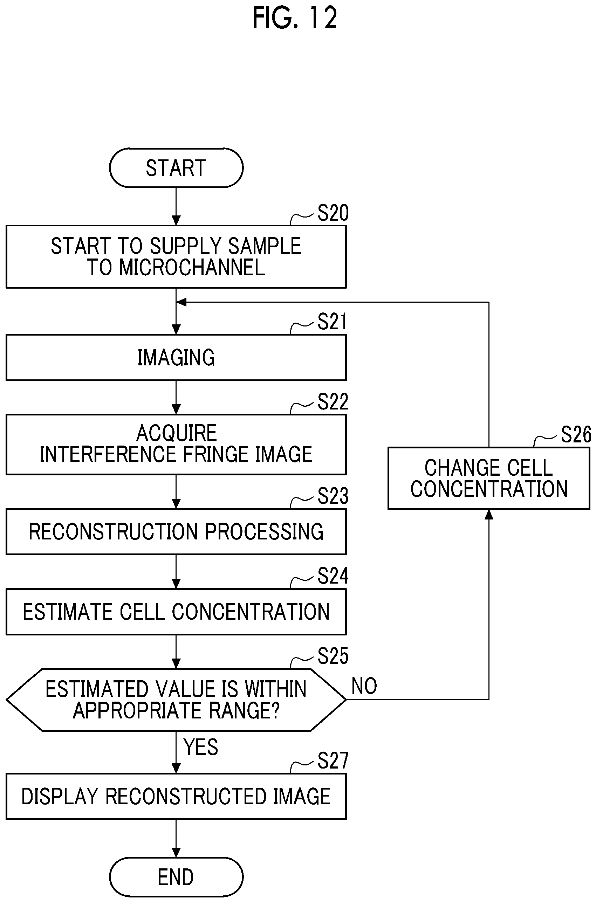

Fig. 12 shows an example of a flow of an operation of the imaging system 4 according to the fourth modification example. Steps S20 to S22 are the same as steps S10 to S12 of the above-described embodiment shown in Fig. 9. In the present modification example, after step S22, the reconstruction processing unit 52 generates the reconstructed image based on the interference fringe image FP (step S23). The cell concentration estimation unit 54 estimates the cell concentration based on the reconstructed image generated by the reconstruction processing unit 52 (step S24). Steps S25 to S27 after step S24 are the same as steps S14, S15, and S17 of the above-described embodiment shown in Fig. 9.

[Fifth Modification Example]

In the above-described embodiment, the concentration control unit 55 adjusts the cell concentration by changing at least one of the diameter D of the suction port 21A, the position of the suction port 21A, or the suction rate V. Alternatively, the concentration control unit 55 may adjust the cell concentration by controlling an injection amount of a diluent injected into the microchannel 9.

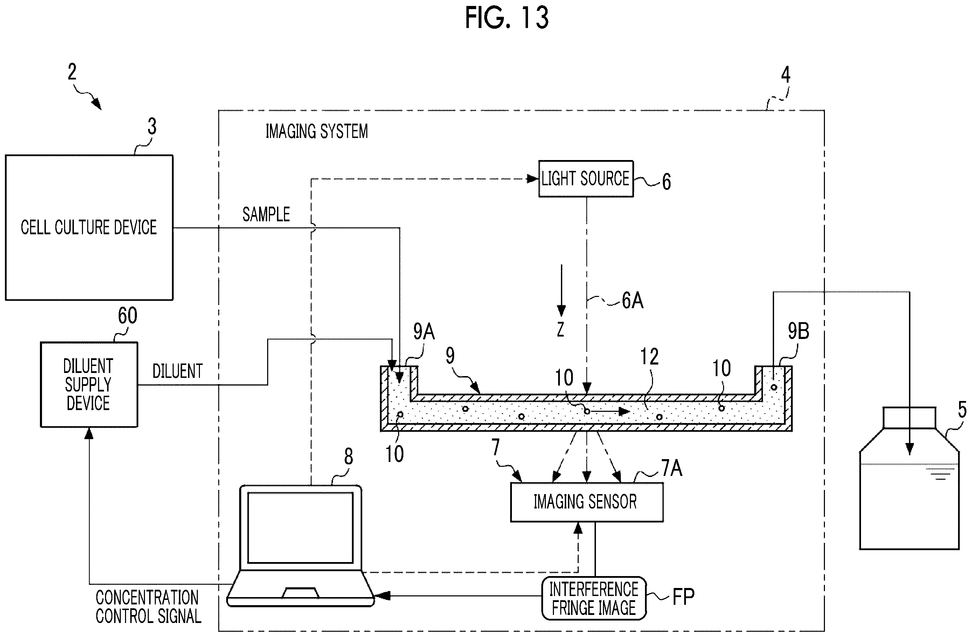

Fig. 13 shows a configuration of the cell culture system 2 according to a fifth modification example. In the present modification example, in addition to the cell culture device 3, the imaging system 4, and the recovery tank 5, a diluent supply device 60 is provided in the cell culture system 2. The diluent supply device 60 injects the diluent into the microchannel 9 from the opening part 9A through a path different from a path for the sample 12 supplied by the cell culture device 3. The diluent is injected into the sample 12 flowing through the microchannel 9, thereby decreasing the cell concentration.
In the present modification example, the concentration control unit 55 adjusts the cell concentration by controlling the diluent supply device 60.

[Sixth Modification Example]

In the above-described embodiment, the concentration control unit 55 repeatedly changes the cell concentration until it is determined that the estimated value R is within the appropriate range. However, the concentration control unit 55 may perform control to notify of an abnormality in a case where the estimated value R is not within the appropriate range even after the cell concentration is changed a certain number of times. This is because it is considered that an abnormality, such as the sample 12 being clogged and not flowing at any location in the flow channel, occurs in a case where the estimated value R is not within the appropriate range even after the cell concentration is changed a certain number of times.

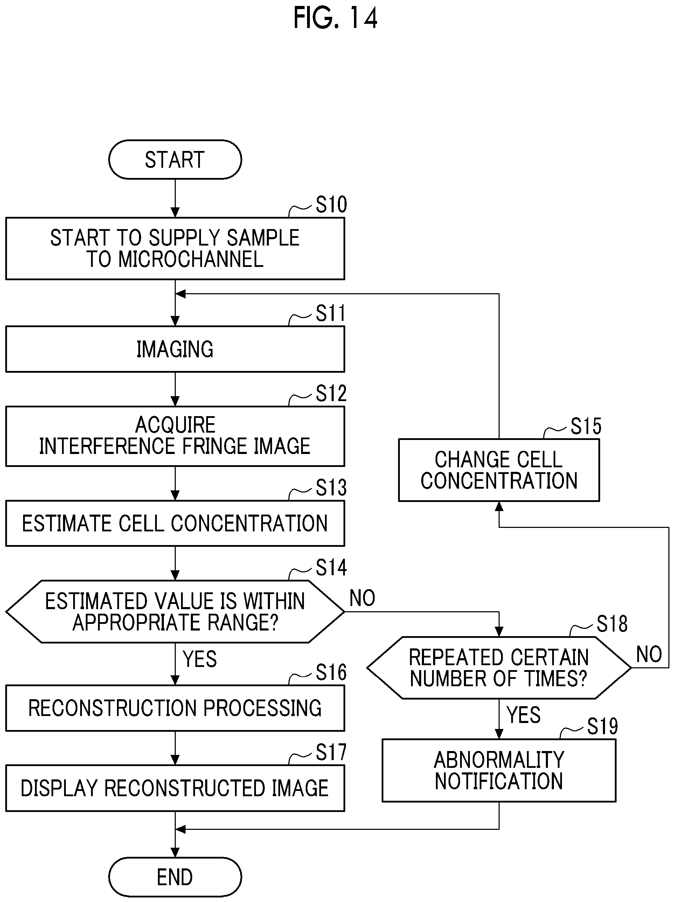

Fig. 14 shows a flow of an operation of the imaging system 4 according to a sixth modification example. In a flowchart shown in Fig. 14, steps S18 and S19 are added to the flowchart shown in Fig. 9. In the present modification example, in a case where it is determined that the estimated value R is not within the appropriate range (step S14: NO), the concentration control unit 55 determines whether or not the change in the cell concentration by step S15 has been repeated a certain number of times (step S18). In a case where it is determined that the change in the cell concentration is not repeated a certain number of times (step S18: NO), the concentration control unit 55 changes the cell concentration (step S15).

On the other hand, in a case where it is determined that the change in the cell concentration has been repeated a certain number of times (step S18: YES), the concentration control unit 55 performs the control to notify of an abnormality (step S19). For example, the concentration control unit 55 controls the display control unit 53 such that the display 44 displays a message notifying a user that an abnormality has occurred.

[Other Modification Examples]

In the above-described embodiment and each of the above-described modification examples, the concentration control unit 55 changes the cell concentration in a direction of decreasing the cell concentration in a case where the estimated value R is higher than the appropriate range, and changes the cell concentration in a direction of increasing the cell concentration in a case where the estimated value R is lower than the appropriate range. Alternatively, the concentration control unit 55 may change the cell concentration in a direction of decreasing the cell concentration only in a case where the estimated value R is higher than the appropriate range.

The imaging system 4 according to the above-described embodiment and each of the above-described modification examples relates to a technology referred to as so-called lens-free imaging in which an optical lens is not provided. The technology of the present disclosure is not limited to the lens-free imaging and can be applied to general digital holography (for example, in a case where reference light is used).

A hardware configuration of a computer constituting the information processing apparatus 8 can be modified in various ways. For example, the information processing apparatus 8 can be configured by a plurality of computers separated as hardware for the purpose of improving processing capacity and reliability.

As described above, the hardware configuration of the computer of the information processing apparatus 8 can be changed as appropriate according to required performance such as processing capacity, safety, and reliability. Further, not only the hardware but also the application program such as the operation program 41A may be duplicated or stored in a plurality of storage devices in a distributed manner for the purpose of ensuring safety and reliability.

In the above-described embodiment and each of the above-described modification examples, for example, as a hardware structure of a processing unit that executes various types of processing, such as the imaging control unit 50, the interference fringe image acquisition unit 51, the reconstruction processing unit 52, the display control unit 53, the cell concentration estimation unit 54, and the concentration control unit 55, various processors shown below can be used. The various processors include a programmable logic device (PLD) which is a processor whose circuit configuration is changeable after manufacturing such as a field programmable gate array (FPGA), a dedicated electric circuit which is a processor having a circuit configuration exclusively designed to execute specific processing such as an application specific integrated circuit (ASIC), and the like, in addition to the CPU 40 which is a general-purpose processor that executes software (operation program 41A) to function as various processing units, as described above.

One processing unit may be configured by one of the various processors, or may be a combination of two or more processors of the same type or different types (for example, a combination of a plurality of FPGAs and/or a combination of a CPU and an FPGA). A plurality of processing units may be configured by one processor.

As an example of configuring a plurality of processing units with one processor, first, there is a form in which, as typified by computers such as a client and a server, one processor is configured by combining one or more CPUs and software, and the processor functions as a plurality of processing units. Second, as typified by a system on chip (SoC) or the like, there is a form in which a processor that realizes functions of an entire system including a plurality of processing units with one integrated circuit (IC) chip is used. As described above, the various types of processing units are configured using one or more of the various types of processors as a hardware structure.

Further, as the hardware structure of the various types of processors, more specifically, an electric circuit (circuitry) in which circuit elements such as semiconductor elements are combined can be used.

The above-described embodiment and each modification example can be combined as appropriate as long as there is no contradiction.

All documents, patent applications, and technical standards described in the present specification are incorporated in the present specification by reference to the same extent as in a case where each document, patent application, and technical standard are specifically and individually noted to be incorporated by reference.

The following technologies can be ascertained by the above description.

[Additional note 1]

An imaging system comprising: a light source that emits light toward a flow channel through which a sample containing a cell flows; an imaging sensor that generates an interference fringe image including an interference fringe by imaging light that has passed through the flow channel; and a processor that estimates a cell concentration in the sample based on the interference fringe image or a reconstructed image obtained by reconstructing the interference fringe image, and adjusts the cell concentration in the sample based on an estimated value of the cell concentration.

[Additional note 2]

The imaging system according to additional note 1, in which the processor estimates the cell concentration based on the number of the interference fringes included in the interference fringe image or frequency information of the interference fringe image.

[Additional note 3]

The imaging system according to additional note 1, in which the processor estimates the cell concentration based on an image quality of the reconstructed image.

[Additional note 4]

The imaging system according to additional note 1, in which the processor estimates the cell concentration based on a brightness or a brightness distribution of the entire interference fringe image.

[Additional note 5]

The imaging system according to any one of additional notes 1 to 4, in which the processor adjusts the cell concentration in the sample by controlling a pump that introduces the sample from a culture tank into the flow channel.

[Additional note 6]

The imaging system according to additional note 5, in which the processor adjusts the cell concentration in the sample by controlling at least one of a diameter of a suction port of the pump, a position of the suction port, or a suction rate.

[Additional note 7]

The imaging system according to any one of additional notes 1 to 4, in which the processor adjusts the cell concentration in the sample by controlling an injection amount of a diluent to be injected into the flow channel.

[Additional note 8]

The imaging system according to any one of additional notes 1 to 7, in which in a case where the estimated value is higher than an appropriate range, the processor changes the cell concentration stepwise until the estimated value is within the appropriate range.

[Additional note 9]

The imaging system according to additional note 8, in which in a case where the estimated value is lower than the appropriate range, the processor changes the cell concentration stepwise until the estimated value is within the appropriate range.

[Additional note 10]

The imaging system according to additional note 8 or 9, in which the processor performs control to notify of an abnormality in a case where the estimated value is not within the appropriate range even after the cell concentration is changed a certain number of times.

[Additional note 11]

A cell concentration adjustment method comprising: emitting light toward a flow channel through which a sample containing a cell flows; generating an interference fringe image including an interference fringe by imaging light that has passed through the flow channel; and estimating a cell concentration in the sample based on the interference fringe image or a reconstructed image obtained by reconstructing the interference fringe image, and adjusting the cell concentration in the sample based on an estimated value of the cell concentration.

**Claims**

1. An imaging system comprising:

   a light source that emits light toward a flow channel through which a sample containing a cell flows;
   an imaging sensor that generates an interference fringe image including an interference fringe by imaging light that has passed through the flow channel; and
   a processor that estimates a cell concentration in the sample based on the interference fringe image or a reconstructed image obtained by reconstructing the interference fringe image, and adjusts the cell concentration in the sample based on an estimated value of the cell concentration.

2. The imaging system according to claim 1,
   wherein the processor estimates the cell concentration based on the number of the interference fringes included in the interference fringe image or frequency information of the interference fringe image.

3. The imaging system according to claim 1,
   wherein the processor estimates the cell concentration based on an image quality of the reconstructed image.

4. The imaging system according to claim 1,
   wherein the processor estimates the cell concentration based on a brightness or a brightness distribution of the entire interference fringe image.

5. The imaging system according to claim 1,
   wherein the processor adjusts the cell concentration in the sample by controlling a pump that introduces the sample from a culture tank into the flow channel.

6. The imaging system according to claim 5,
   wherein the processor adjusts the cell concentration in the sample by controlling at least one of a diameter of a suction port of the pump, a position of the suction port, or a suction rate.

7. The imaging system according to claim 1,
   wherein the processor adjusts the cell concentration in the sample by controlling an injection amount of a diluent to be injected into the flow channel.

8. The imaging system according to claim 1,
   wherein in a case where the estimated value is higher than an appropriate range, the processor changes the cell concentration stepwise until the estimated value is within the appropriate range.

9. The imaging system according to claim 8,
   wherein in a case where the estimated value is lower than the appropriate range, the processor changes the cell concentration stepwise until the estimated value is within the appropriate range.

10. The imaging system according to claim 8 or 9,
    wherein the processor performs control to notify of an abnormality in a case where the estimated value is not within the appropriate range even after the cell concentration is changed a certain number of times.

11. A cell concentration adjustment method comprising:

    emitting light toward a flow channel through which a sample containing a cell flows;

generating an interference fringe image including an interference fringe by imaging light that has passed through the flow channel; and

estimating a cell concentration in the sample based on the interference fringe image or a reconstructed image obtained by reconstructing the interference fringe image, and adjusting the cell concentration in the sample based on an estimated value of the cell concentration.

FIG. 1

IMAGING SYSTEM

LIGHT SOURCE ~6

CELL CULTURE DEVICE

SAMPLE

CONCENTRATION CONTROL SIGNAL

IMAGING SENSOR

INTERFERENCE FRINGE IMAGE ~FP

EP 4 488 662 A1

# FIG. 2

CELL CULTURE DEVICE

INFORMATION PROCESSING APPARATUS

CONTROLLER

SAMPLE

EP 4 488 662 A1

# FIG. 3

## FIG. 4

## FIG. 5

# FIG. 6

# FIG. 7

# FIG. 8

# FIG. 9

```
                    ┌──────────────┐
                    │    START     │
                    └──────┬───────┘
                           │          ~S10
              ┌────────────▼─────────────┐
              │   START TO SUPPLY SAMPLE │
              │      TO MICROCHANNEL     │
              └────────────┬─────────────┘
                           │          ~S11
              ┌────────────▼─────────────┐
              │        IMAGING           │
              └────────────┬─────────────┘
                           │          ~S12
              ┌────────────▼─────────────┐              ~S15
              │        ACQUIRE           │      ┌─────────────────┐
              │ INTERFERENCE FRINGE IMAGE│      │   CHANGE CELL   │
              └────────────┬─────────────┘      │  CONCENTRATION  │
                           │          ~S13      └────────▲────────┘
              ┌────────────▼─────────────┐               │
              │ ESTIMATE CELL CONCENTRATION│             │
              └────────────┬─────────────┘               │
                           │          ~S14               │
                  ╱────────▼─────────╲    NO             │
                 ╱ ESTIMATED VALUE IS  ╲──────────────────┘
                 ╲ WITHIN APPROPRIATE  ╱
                  ╲     RANGE?        ╱
                   ╲────────┬────────╱
                           │ YES
                           │          ~S16
              ┌────────────▼─────────────┐
              │  RECONSTRUCTION PROCESSING│
              └────────────┬─────────────┘
                           │          ~S17
              ┌────────────▼─────────────┐
              │  DISPLAY RECONSTRUCTED IMAGE│
              └────────────┬─────────────┘
                           │
                    ┌──────▼───────┐
                    │     END      │
                    └──────────────┘
```

FIG. 10

# FIG. 11

# FIG. 12

```
          ┌─────────────┐
          │    START    │
          └──────┬──────┘
                 │            ~S20
     ┌───────────▼───────────┐
     │  START TO SUPPLY SAMPLE │
     │     TO MICROCHANNEL     │
     └───────────┬───────────┘
                 │◄─────────────────────────────┐
                 │            ~S21               │
     ┌───────────▼───────────┐                   │
     │        IMAGING        │                   │
     └───────────┬───────────┘                   │
                 │            ~S22               │
     ┌───────────▼───────────┐      ┌────────────┴──────┐ ~S26
     │        ACQUIRE        │      │   CHANGE CELL     │
     │ INTERFERENCE FRINGE IMAGE │  │  CONCENTRATION    │
     └───────────┬───────────┘      └────────────▲──────┘
                 │            ~S23               │
     ┌───────────▼───────────┐                   │
     │ RECONSTRUCTION PROCESSING │               │
     └───────────┬───────────┘                   │
                 │            ~S24               │
     ┌───────────▼───────────┐                   │
     │ ESTIMATE CELL CONCENTRATION │             │
     └───────────┬───────────┘                   │
                 │            ~S25               │
         ◄───────▼────────►    NO                │
        ╱ ESTIMATED VALUE IS WITHIN ╲────────────┘
        ╲   APPROPRIATE RANGE?      ╱
         ◄───────┬────────►
                 │ YES        ~S27
     ┌───────────▼───────────┐
     │ DISPLAY RECONSTRUCTED IMAGE │
     └───────────┬───────────┘
                 │
          ┌──────▼──────┐
          │     END     │
          └─────────────┘
```

# FIG. 13

EP 4 488 662 A1

# FIG. 14

```
                    ┌─────────────┐
                    │    START    │
                    └──────┬──────┘
                           │
                           ▼              ╭─ S10
        ┌──────────────────────────────┐
        │   START TO SUPPLY SAMPLE      │
        │      TO MICROCHANNEL          │
        └──────────────┬───────────────┘
                       │
                       ▼                  ╭─ S11
        ┌──────────────────────────────┐
        │           IMAGING             │
        └──────────────┬───────────────┘
                       │
                       ▼                  ╭─ S12
        ┌──────────────────────────────┐          ╭─ S15
        │         ACQUIRE               │    ┌──────────────────┐
        │  INTERFERENCE FRINGE IMAGE    │    │   CHANGE CELL     │
        └──────────────┬───────────────┘    │  CONCENTRATION    │
                       │                  ╭─ S13 └──────────────────┘
                       ▼
        ┌──────────────────────────────┐
        │   ESTIMATE CELL CONCENTRATION │
        └──────────────┬───────────────┘
                       │                  ╭─ S14
                       ▼
        ╱─────────────────────────────╲  NO
        │  ESTIMATED VALUE IS WITHIN    ├──────┐
        ╲  APPROPRIATE RANGE?          ╱       │        ╭─ S18
         ╲────────────┬───────────────╱        ▼
                  YES │                  ╱──────────────────╲ NO
                      │          ╭─ S16  │ REPEATED CERTAIN  ├──
                      ▼                  ╲ NUMBER OF TIMES? ╱
        ┌──────────────────────────────┐ ╲────────┬───────╱
        │   RECONSTRUCTION PROCESSING   │      YES │    ╭─ S19
        └──────────────┬───────────────┘          ▼
                       │          ╭─ S17  ┌──────────────────┐
                       ▼                  │   ABNORMALITY     │
        ┌──────────────────────────────┐ │  NOTIFICATION     │
        │  DISPLAY RECONSTRUCTED IMAGE  │ └──────────────────┘
        └──────────────┬───────────────┘
                       │
                       ▼
                ┌─────────────┐
                │     END     │
                └─────────────┘
```

## INTERNATIONAL SEARCH REPORT

International application No.

**PCT/JP2023/014105**

### A. CLASSIFICATION OF SUBJECT MATTER

*G01N 21/45*(2006.01)i; *C12M 1/34*(2006.01)i; *C12Q 1/02*(2006.01)i; *G02B 21/36*(2006.01)i
FI:  G01N21/45 A; C12Q1/02; G02B21/36; C12M1/34

According to International Patent Classification (IPC) or to both national classification and IPC

### B. FIELDS SEARCHED

Minimum documentation searched (classification system followed by classification symbols)

G01N21/00-G01N21/61; C12M1/00-C12M3/10; C12Q1/00-C12Q3/00; G02B21/36; G01N15/00-G01N15/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| Y | WO 2014/203322 A1 (HITACHI, LTD.) 24 December 2014 (2014-12-24)<br>paragraphs [0022]-[0038], fig. 1-5 | 1, 3-11 |
| A | | 2 |
| Y | WO 2018/235476 A1 (SONY CORPORATION) 27 December 2018 (2018-12-27)<br>paragraphs [0030]-[0038], [0052]-[0063], [0082]-[0088], [0101]-[0117], fig. 1-13 | 1, 4-11 |
| Y | JP 2020-514704 A (COMMISSARIAT À L'ÉNERGIE ATOMIQUE ET AUX ÉNERGIES ALTERNATIVES) 21 May 2020 (2020-05-21)<br>paragraphs [0033]-[0094], fig. 1-4 | 1, 3, 5-11 |
| A | JP 2017-146696 A (SONY CORPORATION) 24 August 2017 (2017-08-24)<br>entire text, all drawings | 1-11 |
| A | WO 2020/189218 A1 (FUJIFILM CORPORATION) 24 September 2020 (2020-09-24)<br>entire text, all drawings | 1-11 |

☐ Further documents are listed in the continuation of Box C.     ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/014105**

| Patent document cited in search report | | | Publication date (day/month/year) | Patent family member(s) | | | Publication date (day/month/year) |
|---|---|---|---|---|---|---|---|
| WO | 2014/203322 | A1 | 24 December 2014 | (Family: none) | | | |
| WO | 2018/235476 | A1 | 27 December 2018 | US | 2020/0096941 | A1 | |
| | | | | paragraphs [0075]-[0083], [0097]-[0108], [0128]-[0133], [0147]-[0162], fig 1-13 | | | |
| | | | | DE | 112018003193 | T | |
| | | | | CN | 110770333 | A | |
| JP | 2020-514704 | A | 21 May 2020 | WO | 2018/115734 | A1 | |
| | | | | p. 5, line 30 to p. 19, line 22, fig. 1A-4B | | | |
| | | | | EP | 3559631 | A1 | |
| | | | | FR | 3060746 | A1 | |
| JP | 2017-146696 | A | 24 August 2017 | US | 2019/0049897 | A1 | |
| | | | | entire text, all drawings | | | |
| | | | | WO | 2017/141741 | A1 | |
| | | | | DE | 112017000840 | T | |
| WO | 2020/189218 | A1 | 24 September 2020 | US | 2021/0403847 | A1 | |
| | | | | entire text, all drawings | | | |
| | | | | EP | 3943586 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018158947 A